(19) Europäisches Patentamt
European Patent Office
Office européen des brevets

(11) EP 0 822 935 B1

(12) # EUROPÄISCHE PATENTSCHRIFT

(45) Veröffentlichungstag und Bekanntmachung des Hinweises auf die Patenterteilung:
**07.11.2001 Patentblatt 2001/45**

(21) Anmeldenummer: **96919644.3**

(22) Anmeldetag: **29.04.1996**

(51) Int Cl.[7]: **C07D 451/10**, C07D 451/14

(86) Internationale Anmeldenummer:
**PCT/EP96/01779**

(87) Internationale Veröffentlichungsnummer:
**WO 96/33996 (31.10.1996 Gazette 1996/48)**

(54) **VERFAHREN ZUR HERSTELLUNG VON ENANTIOMERENREINEN TROPASÄUREESTERN**
PROCESS FOR PREPARING PURE ENANTIOMERS OF TROPIC ACID ESTERS
PROCEDE DE PREPARATION D'ENANTIOMERES PURS D'ESTERS D'ACIDE TROPIQUE

(84) Benannte Vertragsstaaten:
**AT BE CH DE DK ES FI FR GB GR IE IT LI LU MC NL PT SE**
Benannte Erstreckungsstaaten:
**LT LV SI**

(30) Priorität: **28.04.1995 DE 19515625**

(43) Veröffentlichungstag der Anmeldung:
**11.02.1998 Patentblatt 1998/07**

(73) Patentinhaber:
- **Boehringer Ingelheim Pharma KG**
  **55218 Ingelheim am Rhein (DE)**
  Benannte Vertragsstaaten:
  **BE CH DE DK ES FI FR GR IT LI LU MC NL PT SE AT**
- **Boehringer Ingelheim International GmbH**
  **55218 Ingelheim am Rhein (DE)**
  Benannte Vertragsstaaten:
  **GB IE**

(72) Erfinder: **BANHOLZER, Rolf**
**D-70597 Stuttgart (DE)**

(74) Vertreter: **Laudien, Dieter, Dr.**
**Boehringer Ingelheim GmbH,**
**Binger Strasse 173**
**55216 Ingelheim (DE)**

(56) Entgegenhaltungen:
**FR-A- 1 289 429**

- **SYNTHESIS, 1976, STUTTGART DE, Seiten 311-312, XP002011575 V.A.FUNG ET AL.: "A convenient synthesis of N-CD3 labelled tropine and atropine"**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 822 935 B1

## Beschreibung

Die Erfindung betrifft ein Verfahren, mit dem sich Ester der (+)- und (-)-Tropasäure in hoher Reinheit mit guter Ausbeute herstellen lassen.

Die Aufgabe, enantiomerenreine Ester der (+)- und (-)-Tropasäure synthetisch herzustellen, ist bisher nicht befriedigend gelöst worden. Auch die Enantiomerentrennung von racemischen Tropasäureestern stößt sehr oft auf überraschende experimentelle Schwierigkeiten und läßt sich nicht generell durchführen. Dies gilt besonders für die höher substituierten Homologen und Analogen des Atropins, etwa das N-Isopropylnoratropin, die Vorstufe des Handelsprodukts Ipratropiumbromid.

Die klassische Tropasäureestersynthese nach I. Mamlock und R. Wolffenstein (Ber. dtsch. Chem. Ges. 41, 731 (1908), bei der O-Acetyltropasäurechlorid mit Tropinhydrochlorid umgesetzt wird, führt, möglicherweise wegen der schlechten Löslichkeit des Tropinhydrochlorids, nicht zu befriedigenden Ergebnissen. Auf optisch-aktive Tropasäure wurde das Verfahren nicht angewendet.

Das Verfahren ist problematisch, weil es leicht zu Nebenreaktionen kommen kann. Dies gilt für basische Bedingungen, bei denen die Gefahr besteht, daß Wasser eliminiert wird, andererseits machte die Verwendung von Salzen der Aminoalkohole, die meist schwerlöslich sind, höhere Temperaturen notwendig, die zur Bildung sehr störender Nebenprodukte führen würden; zu nennen sind hier vor allem dehydratisierte Verbindungen (Apoverbindungen) und Dimerisierungsprodukte.

Es wurde nun gefunden, daß die Herstellung enantiomerenreiner (+)- und (-)- Tropasäureester von Aminoalkoholen der Formel

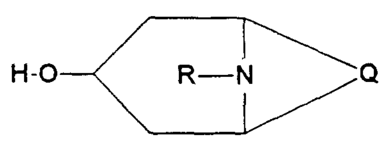

(I)

in der

Q für $CH_2$-$CH_2$, $CH_2$-$CH_2$-$CH_2$, CH=CH oder

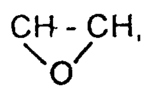

R für eine geradkettige oder verzweigte C1-C4-Alkyigruppe steht,

überraschenderweise gelingt, indem man nach

(a) Acetylierung der entsprechenden optisch-aktiven Tropasäure zur O-Acetyltropasäure und
(b) anschließende Überführung der O-Acetyltropasäure mit Thionylchlorid in das Säurechlorid
das so erhaltene O-Acetyltropasäurechlorid
(c) bei Raumtemperatur in einem inertem Lösungsmittel, gegebenenfalls unter Zusatz überschüssigen Aluminiumoxids, mit dem Methansulfonat eines Aminoalkohols der Formel (I) umsetzt,
(d) die resultierende Verbindung durch Einwirkung einer starken Säure entacetyliert und
(e) den erhaltenen optisch-aktiven Tropasäureester isoliert.

Die als Ausgangsstoff benötigte (+)- und (-)-Tropasäure kann aus D,L-Tropasäure erhalten werden, indem man zunächst in an sich bekannter Weise ein Salz mit einer optisch-aktiven Base herstellt und dieses mehrmals umkristallisiert. Als Base eignet sich z. B. (-)-Chinin, als Lösungsmittel zum Umkristallisieren Ethanol. Die so hergestellte (+)-Tropasäure hat eine Reinheit von 99,8% ($[\alpha]_D^{20}$ = + 73,1; c = 1 in Ethanol).

Die Umsetzung gemäß (a) erfolgt zweckmäßig bei Raumtemperatur, die gleich anschließend durchführbare Umsetzung gemäß (b) wird bevorzugt ohne Zwischenisolierung der acetylierten Säure bei Raumtemperatur oder leicht erhöhter Temperatur vorgenommen.

Stufe (c) verläuft innerhalb einiger Tage, wenn das Methansulfonat einer Verbindung der Formel (I) in einem inerten Lösungsmittel, etwa Methylenchlorid, bei Temperaturen zwischen 0°C und etwa 30°C, vorzugsweise bei Raumtemperatur unter Rühren, zur Reaktion gebracht wird, wobei zum Abfangen der Säure überschüssiges Aluminiumoxid verwendet werden kann. Der Rückstand läßt sich, nach dem Entfernen des Lösungsmittels unter vermindertem Druck, direkt weiterverarbeiten.

in Stufe (d) werden günstige Resultate erhalten, wenn die Entacetylierung mit einer verdünnten wäßrigen Mineralsäure, etwa 2 - 20prozentiger Salzsäure, vorzugsweise 3 - 10prozentiger Salzsäure, bei Raumtemperatur erfolgt. So wird z. B. mit 5prozentiger Salzsäure innerhalb von etwa 2 Tagen eine vollständige Umsetzung erreicht.

Das Reaktionsprodukt kann als Base isoliert werden (Stufe (e)), indem man die saure Reaktionslösung in überschüssige verdünnte (z. B. 20prozentige) Natronlauge oder in wäßrige Alkalicarbonatlösung einrührt und das kristallin ausfallende Produkt abfiltriert. Dabei können Temperaturen zwischen -15 und +50°C angewendet werden; bevorzugt ist die Verwendung von Natriumcarbonatlösung bei etwa 20°C. Aus der jeweiligen Base lassen sich leicht die Salze herstellen, beispielsweise das entsprechende Hydrochlorid, indem man die stöchiometrische Menge etherischer Salzsäure zu der Lösung der Base, z. B. in Methylenchlorid, hinzufügt.

Die auf diese Weise erhaltenen Ester bestehen - entsprechend reine Ausgangssäure vorausgesetzt - zu über 99% aus der reinen optisch-aktiven Verbindung.

Die erfindungsgemäß erhältlichen optisch-aktiven Ester sind wertvolle Zwischenprodukte für die Herstellung der entsprechenden anticholinergen Quartärverbindungen, etwa der entsprechenden Methobromide oder Methomethansulfonate, die beispielsweise als Mittel bei asthmatischen Erkrankungen bzw. obstruktiven Atemwegserkrankungen eingesetzt werden können.

Die Herstellung der Quartärverbindungen nach üblichen Methoden ist ohne Racemisierung möglich.

In den nachstehenden Beispielen ist die erfindungsgemäße Umsetzung näher erläutert, doch sind die erfindungsgemäßen Reaktionsbedingungen nicht auf die konkreten Angaben beschränkt.

Beispiel 1

(-)-Tropasäure-N-isopropylnortropinester-hydrochlorid

(a) 13,3 g (-)-Tropasäure werden unter Rühren bei Raumtemperatur in 31,4 g Acetylchlorid eingetragen, wonach innerhalb einer Stunde eine klare Lösung entsteht. Nach einer weiteren Stunde ist nach dem Dünnschichtchromatogramm (DC) die Umsetzung praktisch vollständig. Zu der (-)-O-Acetyltropasäurelösung werden in 30 Minuten 47,5 g Thionylchlorid zugetropft. Die Lösung wird über Nacht bei Raumtemperatur gerührt, danach noch 1 Stunde bei 50°C. Nach dem Eindampfen bei 35°C unter vermindertem Druck hinterbleiben 18,9 g einer bräunlichen Flüssigkeit, $[\alpha]_D^{20}$ = - 87,8 (c = 0,5 in Chloroform). Das Vorliegen der gewünschten Verbindung läßt sich spektroskopisch bestätigen.

(b) 7,26 g (0,0275 mol) N-Isopropylnortropin-methansulfonat und 6,20 g (0,0275 mol) des nach (a) erhaltenen Produkts werden in 45 ml Methylenchlorid bei Raumtemperatur gerührt. Nach sieben Tagen wird die Reaktionslösung bei 30°C unter vermindertem Druck eingedampft. Der Rückstand (16,9 g) wird ohne weitere Aufarbeitung für die nächste Stufe verwendet.

(c) 11,1 g des Produkts gemäß (b) werden in 60 ml 5prozentiger Salzsäure gelöst und 2 Tage bei Raumtemperatur gerührt. Danach läßt das DC eine quantitative Entacetylierung erkennen.
Die Reaktionslösung wird zweimal mit wenig Diethylether extrahiert, dann werden unter vermindertem Druck Reste des Ethers entfernt. In die Lösung wird nun überschüssige 20prozentige wäßrige Natriumcarbonatlösung eingerührt. dabei fällt ein kristallines Produkt aus. Es wird abfiltriert und mit kaltem Wasser gewaschen, bis das ablaufende Filtrat nur noch schwach alkalische Reaktion zeigt, und in Methylenchlorid gelöst. Nach dem Trocknen über Natriumsulfat wird das Lösungsmittel unter vermindertem Druck abdestilliert und der Rückstand aus Acetonitril umkristallisiert. Man erhält weiße Kristalle, Fp. 131°C, $[\alpha]_D^{20}$ = - 19,1 (c=1 in Ethanol).
Zur Herstellung des Hydrochlorids wird die Lösung der Base in Methylenchlorid mit der stöchiometrischen Menge etherischer Salzsäure versetzt. Nach dem Umkristallisieren des Produkts aus Ethanol und aus Acetonitril liegt das (-)-Tropasäure-N-isopropylnortropinester-hydrochlorid in Form weißer Kristalle, Fp. 214-8°C, vor.
$[\alpha]_D^{20}$ = - 27,8 (c=1 in Wasser); optische Reinheit >99,8%.

Das Vorliegen der Verbindung wurde elementaranalytisch und spektroskopisch bestätigt.

Die Quaternierung zum Methobromid gelingt beispielsweise durch Umsetzung mit Methylbromid in Methylenchlorid/Acetonitril bei Raumtemperatur. Es werden weiße Kristalle erhalten (Ausbeute 75,2% d. Th., Fp. 238-42°C unter Zersetzung).
$[\alpha]_D^{20}$ = - 24,5 (c = 1 in Wasser); optische Reinheit > 99,5%.
Das Vorliegen der Verbindung wurde elementaranalytisch und spektroskopisch bestätigt.

Beispiel 2

(+)-Tropasäure-N-isopropylnortropinester-hydrochlorid

Ausgehend von (+)-Tropasäure, die durch Racematspaltung aus D,L-Tropasäure mit (-)-Chinin erhalten werden kann [a] = + 73,1 (c = 1 in Ethanol; optische Reinheit 99,8%) erhält man analog Beispiel 1 die Titelverbindung, weiße Kristalle, Fp. 214-7°C unter Zersetzung, $[\alpha]_D^{20}$ = + 27,8 (c = 1 in Wasser). Ausbeute 55,7% d. Th.. Auch hier wird das Vorliegen der Verbindung elementaranalytisch und spektroskopisch bestätigt.

Wie in Beispiel 1 beschrieben, wird die erhaltene Verbindung zum Methobromid umgesetzt; Fp. 238-41°C (Zers.), $[\alpha]_D^{20}$ = + 25,3 (c = 1 in Wasser).

Für die Umsetzung der O-Acetyltropasäurechloride mit den Verbindungen (I) werden jeweils Ausbeuten zwischen etwa 60 und 70 % erhalten, wenn die Umsetzungen bei Raumtemperatur über einige Tage laufen, wobei neben Methylenchlorid z. B. auch Dimethylformamid oder Acetonitril als Reaktionsmedium ähnliche Ergebnisse liefern.

**Patentansprüche**

1. Verfahren zur Herstellung enantiomerenreiner (+)- und (-)-Tropasäureester von Aminoalkoholen der Formel

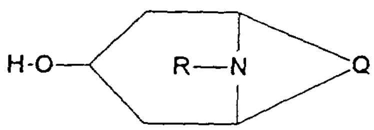

(I)

in der

Q für $CH_2$-$CH_2$, $CH_2$-$CH_2$-$CH_2$, CH=CH oder

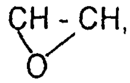

R für eine geradkettige oder verzweigte $C_1$-$C_4$-Alkylgruppe steht,
durch Umsetzung eines Aminoalkohols der Formel (I) mit O-Acetyltropasäurechlorid, erhältlich durch

(a) Acetylierung der entsprechenden optisch-aktiven Tropasäure zur O-Acetyltropasäure und
(b) anschließende Überführung der O-Acetyltropasäure mit Thionylchlorid in das Säurechlorid,

**dadurch gekennzeichnet, daß** man

(c) O-Acetyltropasäurechlorid bei Raumtemperatur in einem inerten Lösungsmittel mit dem Methansulfonat eines Aminoalkohols der Formel (I) umsetzt,
(d) die entstandene Verbindung durch Einwirkung einer starken Säure entacetyliert und
(e) den erhaltenen optisch-aktiven Tropasäureester isoliert.

2. Verfahren nach Anspruch 1, **dadurch gekennzeichnet, daß** die Umsetzung gemäß Schritt (c) der Reaktionsfolge in Gegenwart überschüssigen Aluminiumoxids durchgeführt wird.

3. Verfahren nach Anspruch 1, **dadurch gekennzeichnet, daß** im Schritt (e) der Reaktionsfolge der optisch-aktive Tropasäureester aus der sauren Lösung mit wäßriger Natriumcarbonatlösung ausgefällt wird.

4. Verfahren nach Anspruch 1 bis 3, **dadurch gekennzeichnet, daß** als Verbindung der Formel (1) N-Isopropylnortropinmethansulfonat eingesetzet wird.

## Claims

1. Process for preparing enantiomerically pure (+)- and (-)-tropic acid esters of amino alcohols of formula

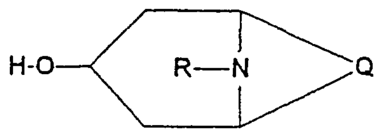

(I)

wherein

Q denotes $CH_2$-$CH_2$, $CH_2$-$CH_2$-$CH_2$, CH=CH or

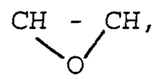

R denotes a straight-chained or branched $C_{1-4}$-alkyl group,

by reacting an aminoalcohol of formula (I) with O-acetyltropic acid chloride, obtainable by

(a) acetylating the corresponding optically active tropic acid to obtain O-acetyl tropic acid and

(b) subsequently converting the O-acetyl tropic acid with thionyl chloride into the acid chloride,

**characterised in that**

(c) O-acetyl tropic acid chloride is reacted at ambient temperature in an inert solvent with the methanesulphonate of an aminoalcohol of formula (I) ,
(d) the resulting compound is deacetylated by the action of a strong acid and
(e) the optically active tropic acid ester obtained is isolated.

2. Process according to claim 1, **characterised in that** the reaction according to step (c) of the reaction sequence is carried out in the presence of excess aluminium oxide.

3. Process according to claim 1, **characterised in that** in step (e) of the reaction sequence the optically active tropic acid ester is precipitated from the acid solution using aqueous sodium carbonate solution.

4. Process according to claims 1 to 3, **characterised in that** N-isopropylnortropine methanesulphonate is used as the compound of formula (I).

## Revendications

1. Procédé de préparation d'esters énantiomériquement purs d'acide (+) et (-)-tropique et d'aminoalcools de formule

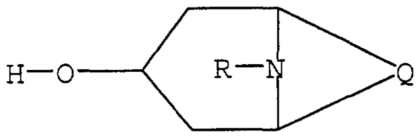

(I)

où

Q représente $CH_2$-$CH_2$, $CH_2$-$CH_2$-$CH_2$, CH=CH ou

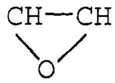

R représente un groupe alkyle en $C_1$-$C_4$ linéaire ou ramifié,
par réaction d'un aminoalcool de formule (I) avec le chlorure d'acide O-acétyltropique, qui peut être obtenu par

(a) acétylation de l'acide tropique optiquement actif correspondant en acide O-acétyltropique et
(b) conversion subséquente de l'acide O-acétyltropique en le chlorure d'acide avec le chlorure de thionyle,

**caractérisé en ce que**

(c) on fait réagir le chlorure d'acide O-acétyltropique à la température ambiante dans un solvant inerte avec le méthanesulfonate d'un aminoalcool de formule (I),
(d) on désacétyle le composé formé en faisant agir un acide fort et
(e) on isole l'ester d'acide tropique optiquement actif obtenu.

2. Procédé selon la revendication 1 **caractérisé en ce que** la réaction selon l'étape (c) de la suite de réactions est conduite en présence d'oxyde d'aluminium en excès.

3. Procédé selon la revendication 1 **caractérisé en ce que**, dans l'étape (e) de la suite de réactions, l'ester d'acide tropique optiquement actif est précipité dans la solution acide avec une solution aqueuse de carbonate de sodium.

4. Procédé selon les revendications 1 à 3 **caractérisé en ce que** le méthanesulfonate de N-isopropylnortropine est utilisé comme composé de formule (I).